# EUROPEAN PATENT APPLICATION

(11) **EP 2 236 111 A1**
(43) Date of publication of application: **06.10.2010**
(21) Application number: 07860558.1
(22) Date of filing: 25.12.2007
(51) Int. Cl.: A61F 13/15, A61F 13/49

(54) **TRUNKS TYPE DIAPER**

(71) Applicant: Koyo Corporation, Kanagawa 247-0014 (JP)
(72) Inventor: SHIMEZAKI, Yukio, Yokohama-shi Kanagawa 236-0004 (JP); TAKAGI, Takamasa, Yokohama-shi Kanagawa 236-0004 (JP); MITSUGI, Takehisa, Yokohama-shi Kanagawa 236-0004 (JP); MATSUMIYA, Munetada, Yokohama-shi Kanagawa 236-0004 (JP)
(74) Representative: Görz, Ingo
(86) International application number: PCT/JP2007/075363
(87) International publication number: WO 2009/081505

(57) **Abstract**

The present invention aims at providing a trunks type diaper which allows a good feeling of wearing around both thighs when wearing the diaper, is easily worn and undressed, and has high leakage prevention.

In a situation where the diaper is developed, a first thigh elastic member (15a) extends substantially linearly from a left edge (7a1) of the front main section to a right edge (7b1) of the front main section via a crotch section (6) front edge; a second thigh elastic member (15b) also extends substantially linearly from a left edge (7a2) of the rear main section to a right edge (7b2) of the rear main section via a crotch section (6) rear edge. As a result, in the bottom of the diaper in a situation where the left and right edges (7a1, 7b1, 7a2, 7b2) of the front and rear main sections (2, 3) are joined, the first and second thigh elastic members are arranged to surround the left and right thigh openings (5a, 5b) as one pair.

## Description

### Technical Field

The present invention relates to a trunks type diaper, more particularly to a trunks type diaper which allows a good feeling of wearing around both thighs when putting the diaper on, has high leakage prevention, and has stable absorptivity.

### Background Art

The trunks type diaper is widely used for an infant, an incontinent adult, an elderly person, etc. It is desirable that such a trunks type diaper allows a good feeling of wearing, the stable absorptivity of bodily waste, and high leakage prevention. Thus, various attempts have been carried out to improve. One diaper is proposed in which elastic members are provided at thigh opening peripheral edges to provide leg gathers so that the lateral leakage of bodily waste is prevented.

Various arrangements of the elastic members for obtaining these leg gathers are proposed. For example, Japanese Patent Application Publication No. H3-280951 and Japanese Patent Application Publication No. H3-186263 disclose one in which the first and second thigh elastic members are arranged to be crossed in two positions at a crotch section, and Japanese Registered Utility Model No. 3024357 discloses one in which first and second elastic threads are not crossed at the crotch section but arranged to be brought into the closest proximity with each other.

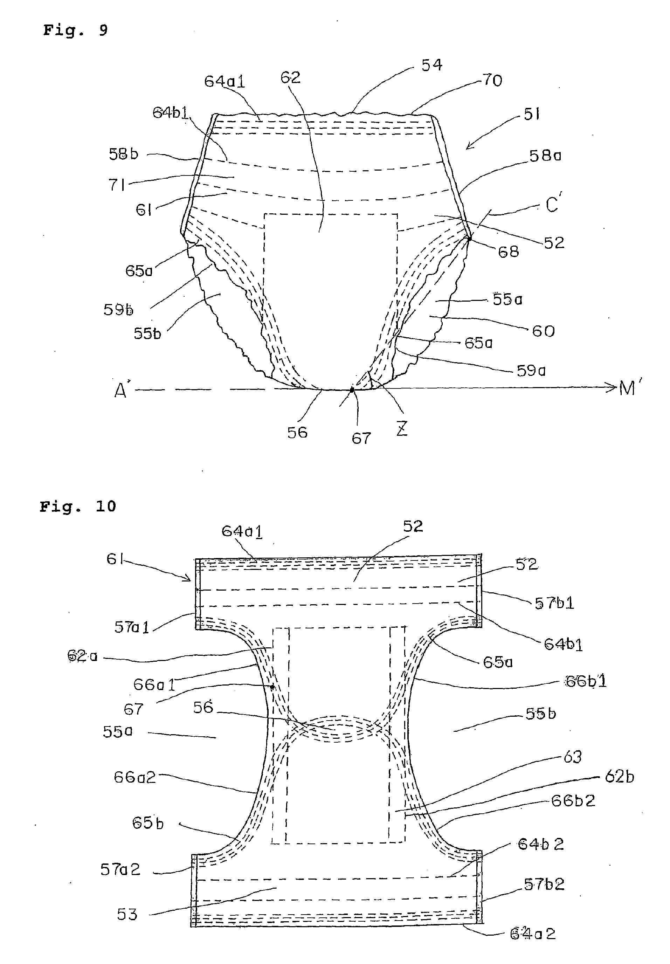

Further, FIGS. 9 through 11 show more particularly one in which the above-mentioned first and second thigh elastic members are crossed in two positions at the crotch section. FIGS. 9, 12, and 13 show one in which they are brought into the closest proximity with each other. Referring to these drawings, a diaper 51 is constituted by a back sheet 61, an absorber 62 in which solid gathers 63 are formed at side edges, the absorber being provided at an internal surface of the back sheet 61, waist elastic members 64a1 and 64a2, torso elastic members 64b1 and 64b2, and first and second thigh elastic members 65a and 65b.

From a developed state of the diaper as shown in FIG. 10, the diaper is folded to form a front main section 52, a rear main section 53, and a crotch section 56. By mutually joining left and right edges 57a1 and 57b1 of the front main section 52 to left and right edges 57a2 and 57b2 of the rear main section 53 side by side, junction parts 58a and 58b in the right side and left side of the diaper 51 are formed to form the diaper 51 as shown in FIG. 9. The diaper 51 is provided with a waist opening 54 and the left and right thigh openings 55a and 55b in order that a user may put on and take off the diaper. A plurality of elastic members are provided in a circumferential direction in order to improve fitness of the diaper with respect to a body and in order to prevent the leakage of urine etc. A plurality of the elastic members are each arranged at the waist opening 54, the front main section 52, the rear main section 53, the left and right thigh openings 55a and 55b peripheral edges through the crotch section 56 in each circumferential direction, by means of which waist gathers 70, body gathers 71, and left and right leg gathers 59a and 59b are respectively formed around the waist, around the torso, and around thighs.

Paying attention to the above-mentioned left and right leg gathers 59a and 59b, a plurality of elastic members arranged at the left and right thigh opening peripheral edges through the crotch section are constituted by the first and second thigh elastic members 65a and 65b.

In a situation where the diaper is developed, the first thigh elastic member 65a extends from the left edge 57a1 of the front main section 52, along a front side edge 66a1 of the left thigh opening, through the center of the crotch section 56, along a front side edge 66b1 of the right thigh opening, to the right edge 57b1 of the front main section 52. On the other hand, the second thigh elastic member 65b extends from the left edge 57b2 of the rear main section 53, along a rear side edge 66a2 of the left thigh opening, through the center of the crotch section 56, along a rear side edge 66b2 of the right thigh opening, to the rear side edge 57b2 of the rear main section 53.

In this case, a development view of a pants type diaper in FIG. 10 shows an arrangement of the thigh elastic members in which the first thigh elastic member 65a and the second thigh elastic member 65b are crossed in two positions at the crotch section.

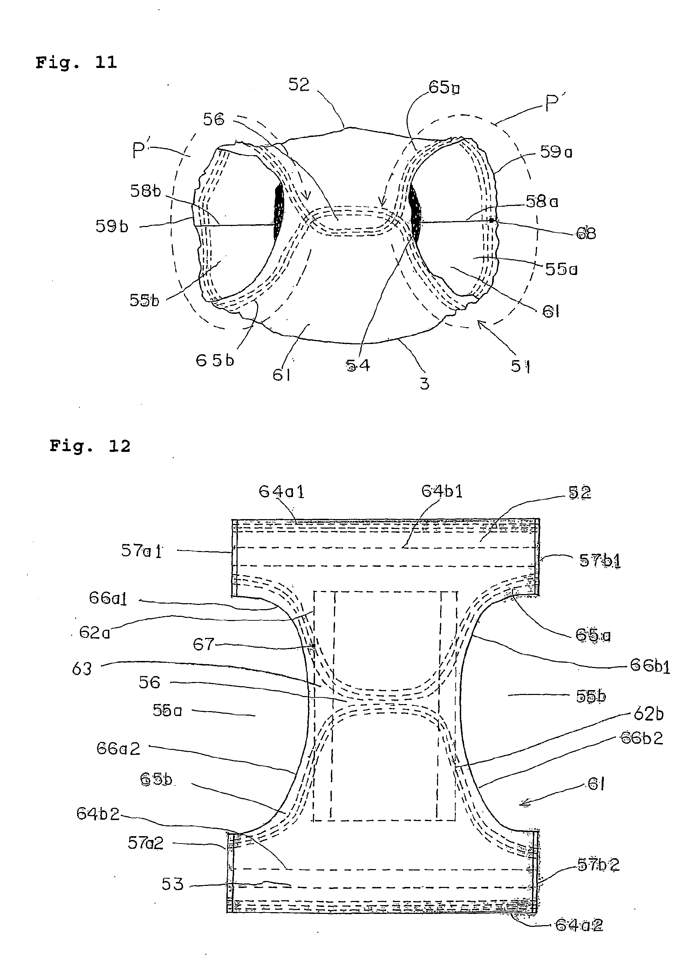

As another prior art, a development view of the pants type diaper in FIG. 12 shows an arrangement of the thigh elastic members in which the first thigh elastic member 65a and the second thigh elastic member 65b are not crossed at the crotch section but brought into the closest proximity with each other.

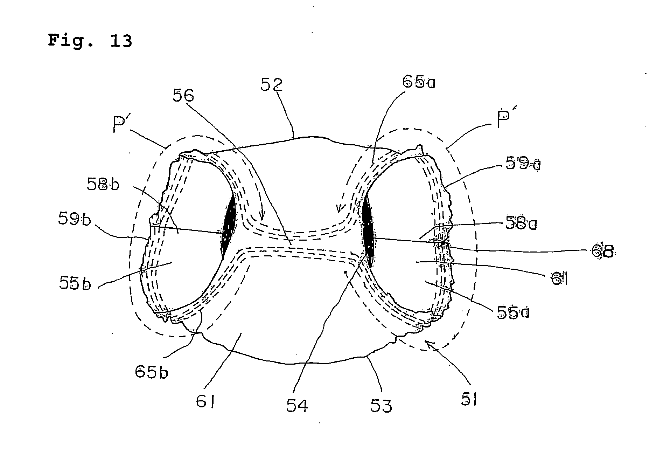

FIG. 11 shows a bottom view of the diaper with the arrangement of the thigh elastic members as in the former. FIG. 13 shows a bottom view of the diaper with the arrangement of the thigh elastic members as in the latter.

An arrangement of these thigh elastic members is such that the thigh elastic members are arranged along the side edges of the thigh openings 55a and 55b via the crotch section 56 center in a situation where the diaper is developed, in order to avoid the generation of a gap between a human's thigh and the thigh opening peripheral edge around the thigh and to arrange the thigh elastic members along the thigh opening peripheral edges.

Further, in order to avoid the generation of a gap when the crotch section 56 is away from the body, the first and second thigh elastic members 65a and 65b are crossed or brought into the closest proximity with each other at the crotch section 56 center in a situation where the diaper is developed, so that the absorber is pressed towards the wearer's skin side by means of an elastic force of the thigh elastic member. In other words, in a situation where the front and rear main sections 52 and 53 are joined together at the left and right edges 57a1, 57a2, 57b1, and 57b2 to form the diaper, the first thigh elastic member 65a surrounds the left and right thigh openings 55a and 55b in the shape of a ring as shown by a curve P' . On the other hand, the second thigh elastic member 65b also surrounds the left and right thigh openings 55a and 55b in the shape of a ring as shown by the curve P'. In other words, the left and right of the first and second thigh elastic members each independently surround the thigh openings in the shape of a ring.

Furthermore FIG. 9 shows an imaginary horizontal line A' which passes through a junction point 67 between a left side edge 62a of an absorber 63 and a first thigh elastic member 65a, as well as an imaginary straight line C' which passes through the above-mentioned junction point 67 and an intersection 68 between the first thigh elastic member 65a and the left edge 57a1 of the front main section 52. As a result, an angle Z of the imaginary straight line C' relative to the imaginary horizontal line A' in the prior art diaper illustrated in FIG. 9 is often set to 45 degrees or more. The same applies not only to the left thigh side of the diaper as described above but also to the right thigh side of the diaper.

Still further, besides the above-mentioned arrangements of the elastic members, there are some diapers in which the first and second thigh elastic members are arranged along the thigh opening peripheral edges but not arranged in the center of the crotch section, as disclosed in Japanese Patent Application Publication No. H6-327716 or Japanese Patent Application Publication No. 2003-102780. Also, in these arrangements, the left and right of the first and second thigh elastic members each independently surround the left and right thigh opening peripheral edges in the shape of a ring.

These conventional diapers suffer from the following problems:
(1) in the crotch section, the first and second thigh elastic members surround each thigh opening in the shape of a ring, so that the absorber may be tangled in the ring at the left and right thigh openings and likely to be in the shape of a string or strap. Further, since the above-mentioned angle Z is 45 degrees or more, if a shrinkage force of the elastic members in the shape of a ring is applied to the absorber, then the absorber may be stretched vertically. Therefore, the absorber tends to be shrunk into the shape of a string or strap, giving the wearer displeasure, generating a gap between the thigh opening peripheral edge and the thigh, which may cause the bodily waste to leak laterally. Further, the stable absorption by the absorber may become difficult.
(2) Since the left and right thigh openings each shrink into the shape of a ring, it may be hard to insert and pull out a leg when putting on or taking off the diaper. In other words, according to prior art, since the left and right thigh openings each shrink into the shape of a ring, it is necessary to expand the diaper laterally not with one hand but with both hands in the case of expanding the shrinkage of the thigh opening when putting on or taking off the diaper. Therefore, in the case of inserting the leg when putting the diaper on, the leg tends to be caught unless the legs are inserted one by one, and it is very hard to put it on. Thus, especially, it is difficult for an old person with trouble in one hand etc. to change the diapers.
(3) Since the left and right thigh openings are each in the shape of a ring, the thighs are tightened when wearing, resulting in impression marks caused by the elastic members, or in poor blood circulation.

Therefore, the present invention aims at solving the above-mentioned problems at a time, and providing a trunks type diaper which allows a good feeling of wearing around both thighs when wearing the diaper, is easily put on and taken off, and has high leakage prevention and stable absorptivity.

### DISCLOSURE OF THE INVENTION

According to a first aspect of the present invention, there is provided a trunks type diaper which is constituted by a back sheet and an absorber, wherein a front main section, a rear main section, a crotch section, a waist opening, and left and right thigh openings are formed, and wherein thigh elastic members are arranged at left and right thigh opening peripheral edges to form left and right leg gathers, **characterized in that** the above-mentioned thigh elastic members are constituted by first and second thigh elastic members, in a situation where each junction between left or right edge of the above-mentioned front main section and left or right edge of the above-mentioned rear main section is separated to develop the diaper, the above-mentioned first thigh elastic member extends substantially linearly from the left edge of the front main section to the right edge of the front main section via a crotch section front edge, the above-mentioned second thigh elastic member also extends substantially linearly from the left edge of the rear main section to the right edge of the rear main section via a crotch section rear edge, in the bottom of the diaper in a situation where the left and right edges of the above-mentioned front main section are joined to the left and right edges of the above-mentioned rear main section to form the trunks type diaper, the above-mentioned first and second thigh elastic members are arranged to surround the left and right thigh openings as one pair, and an angle of an imaginary straight line which passes through a junction point and an intersection between the above-mentioned first thigh elastic member and the left or right edge of the front main section relative to an imaginary horizontal line which passes through the above-mentioned junction point between left or right side edge of the above-mentioned absorber and the above-mentioned first thigh elastic member is set as 0 to 30 degrees.

According to a second aspect of the present invention, there is provided a trunks type diaper, **characterized in that** in a situation where each junction between the left or right edge of the above-mentioned front main section and the left or right edge of the above-mentioned rear main section is separated to develop the diaper, the above-mentioned first thigh elastic member extends linearly from the left edge of the front main section to the right edge of the front main section via the crotch section front edge, the above-mentioned second thigh elastic member also extends linearly from the left edge of the rear main section to the right edge of the rear main section via the crotch section rear edge, in the bottom of the above-mentioned diaper in a situation where the left and right edges of the above-mentioned front main section are joined to the left and right edges of the above-mentioned rear main section to form the trunks type diaper, the above-mentioned first and second thigh elastic members are arranged to surround the left and right thigh openings as one pair, and the angle of the imaginary straight line which passes through the junction point and the intersection between the above-mentioned first thigh elastic member and the left or right edge of the front main section relative to the imaginary horizontal line which passes through the above-mentioned junction point between the left or right side edge of the above-mentioned absorber and the above-mentioned first thigh elastic member is set to 0 degree.

Further, according to a third aspect of the present invention, there is provided a trunks type diaper, **characterized in that** in a situation where each junction between the left or right edge of the above-mentioned front main section and the left or right edge of the above-mentioned rear main section is separated to develop the diaper, a left part of the above-mentioned first thigh elastic member extends substantially linearly in the width direction from the left edge of the front main section to and beyond a left side edge of the absorber, a right part of the above-mentioned first thigh elastic member extends substantially linearly in the width direction from the right edge of the front main section to and beyond a right side edge of the absorber, a left part of the above-mentioned second thigh elastic member extends substantially linearly in the width direction from the left edge of the rear main section to and beyond the left side edge of the absorber, a right part of the above-mentioned second thigh elastic member extends substantially linearly in the width direction from the right edge of the rear main section to and beyond the right side edge of the absorber, in the bottom of the above-mentioned diaper in a situation where the left and right edges of the above-mentioned front main section are joined to the left and right edges of the above-mentioned rear main section to form the trunks type diaper, the above-mentioned first and second thigh elastic members are arranged to surround the left and right thigh openings as one pair, and the angle of the imaginary straight line which passes through the junction point and the intersection between the above-mentioned first thigh elastic member and the left or right edge of the front main section relative to the imaginary horizontal line which passes through the above-mentioned junction point between the left or right side edge of the above-mentioned absorber and the above-mentioned first thigh elastic member is set as 0 to 30 degrees.

Furthermore, according to a fourth aspect of the present invention, there is provided a trunks type diaper, **characterized in that** in a situation where each junction between the left or right edge of the above-mentioned front main section and the left or right edge of the above-mentioned rear main section is separated to develop the diaper, the left part of the above-mentioned first thigh elastic member extends linearly in the width direction from the left edge of the front main section to and beyond the left side edge of the absorber, the right part of the above-mentioned first thigh elastic member extends linearly in the width direction from the right edge of the front main section to and beyond the right side edge of the absorber, the left part of the above-mentioned second thigh elastic member extends linearly in the width direction from the left edge of the rear main section to and beyond the left side edge of the absorber, the right part of the above-mentioned second thigh elastic member extends linearly in the width direction from the right edge of the rear main section to and beyond the right side edge of the absorber, in the bottom of the above-mentioned diaper in a situation where the left and right edges of the above-mentioned front main section are joined to the left and right edges of the above-mentioned rear main section to form the trunks type diaper, the above-mentioned first and second thigh elastic members are arranged to surround the left and right thigh openings as one pair, and the angle of the imaginary straight line which passes through the junction point and the intersection between the above-mentioned first thigh elastic member and the left or right edge of the front main section relative to the imaginary horizontal line which passes through the above-mentioned junction point between the left or right side edge of the above-mentioned absorber and the above-mentioned first thigh elastic member is set to 0 degree.

With the arrangement as described above, in the crotch section, the first and second thigh elastic members surround both the left and right thigh openings into the shape of a round as a whole so as to stretch the absorber in a width direction and the shrinkage force of the thigh elastic member may stretch the absorber in the width direction since the above-mentioned angle is 30 degrees or less. Therefore, the absorber is prevented from being in the shape of a string or a strap, so that wearer discomfort may be alleviated, a gap between the thigh opening peripheral edge and the thigh may be prevented, and it is possible to prevent the bodily waste from leaking laterally.

Further, since the left and right thigh openings are surrounded in the shape of a round as one pair, the problem that it is hard to insert or pull out a leg when putting on or taking off the diaper is improved. In other words, since the left and right thigh openings are surrounded in the shape of a round as one pair, the left and right thigh openings are stretched out at the same time as the diaper is opened with both arms leftward and rightward. Therefore, unlike the prior art, it is possible to reduce the time and effort of inserting each leg into the diaper when putting the diaper on. In other words, as to the conventional diaper in which the left and right thigh openings shrink into the shape of a ring independently, it is impossible to provide the openings as required for inserting the thighs in the case of putting on the diaper unless the diaper is opened laterally with both hands. However, according to the diaper in accordance with the present invention and to the above-described structure, the openings through which the legs are inserted can be obtained by a single hand by holding a part of the diaper (for example, part of leg gathers or absorber) in position. This means that it is possible for an old person with trouble in one hand etc. to change the diapers easily, which is considerable improvement, and provide an effect of increasing the opportunity to be able to put on the diaper alone and independently.

Furthermore, since the left and right thigh openings are in the shape of a round as one pair, the thighs are not bound tight when wearing. There is no impression mark caused by tightening the elastic member etc., leading to good blood circulation of the thighs.

### BRIEF DESCRIPTION OF THE DRAWINGS

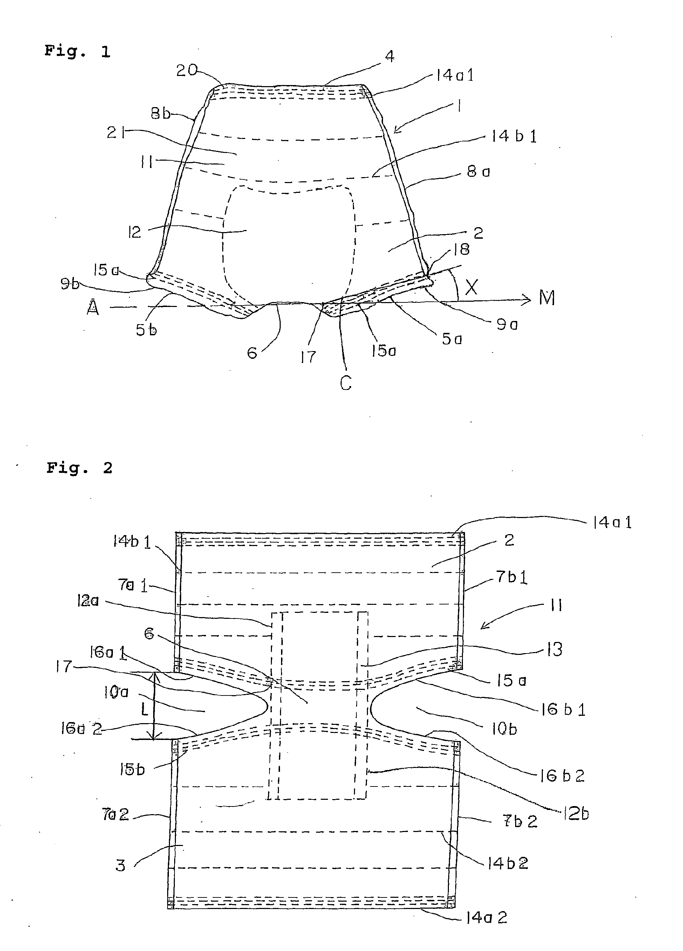

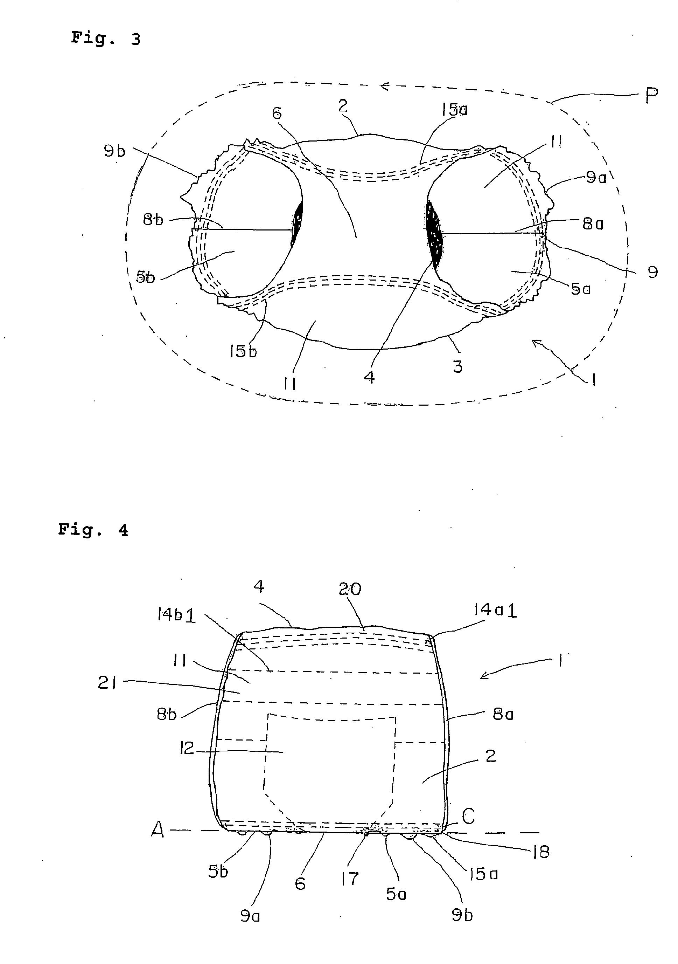

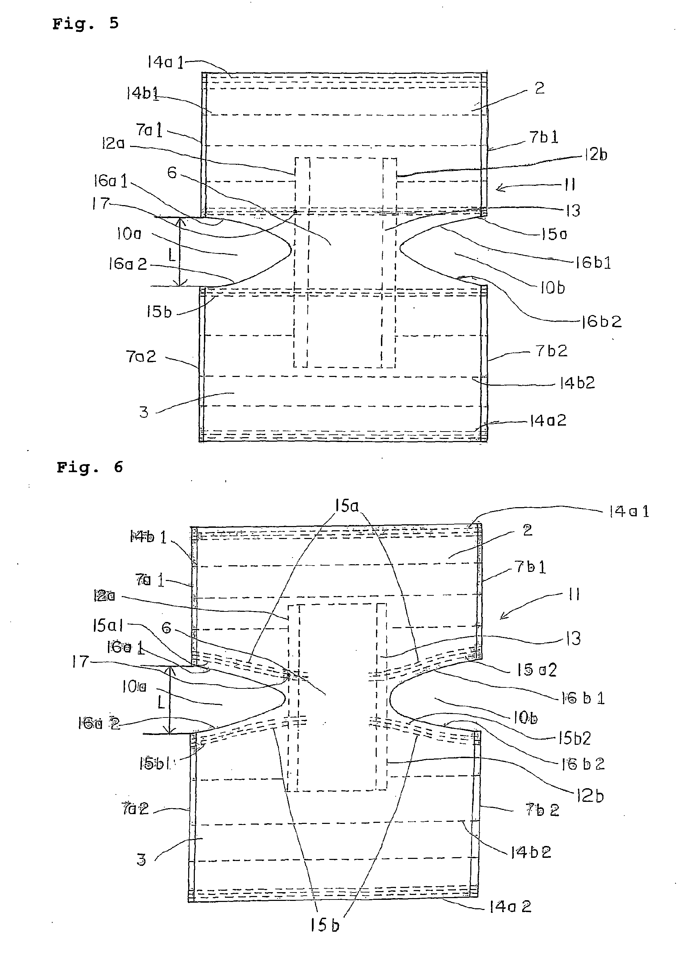

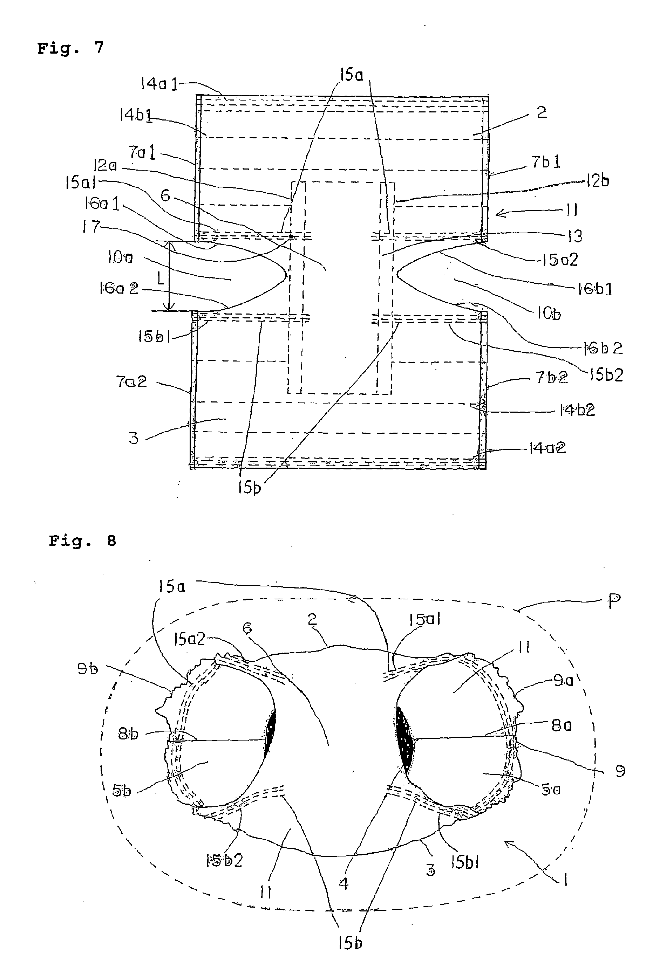

FIG. 1 is a front view of a first preferred embodiment of a trunks type diaper in accordance with the present invention. FIG. 2 is a development view of the trunks type diaper of the first preferred embodiment. FIG. 3 is a bottom view in the first preferred embodiment. FIG. 4 is a front view of a second preferred embodiment of the trunks type diaper in accordance with the present invention. FIG. 5 is a development view of the trunks type diaper of the second preferred embodiment. FIG. 6 is a development view of a third preferred embodiment of the trunks type diaper in accordance with the present invention. FIG. 7 is a development view of a fourth preferred embodiment of the trunks type diaper in accordance with the present invention. FIG. 8 is a bottom view of the trunks type diaper as shown in FIG. 6. FIG. 9 is a front view of a diaper of the prior art. FIG. 10 is a development view of the diaper of the prior art as shown in FIG. 9. FIG. 11 is a bottom view of the diaper of the prior art as shown in FIG. 9. FIG. 12 is a development view of another diaper of the prior art. FIG. 13 is a bottom view of the another diaper of the prior art as shown in FIG. 12.

### BEST MODE FOR CARRYING OUT THE INVENTION

The preferred embodiments of the present invention will be described with reference to FIGS. 1 through 8.

Firstly, a first preferred embodiment of the present invention will be described with reference to FIGS. 1 through 3. A trunks type diaper 1 is constituted by a back sheet 11, an absorber 12, waist elastic members 14a1 and 14a2, torso elastic members 14b1 and 14b2, and first and second thigh elastic members 15a and 15b. The above-mentioned absorber 12 is arranged at an internal surface of the back sheet 11, solid gathers 13 formed of a nonwoven fabric and an elastic material being provided at side edges, and is constituted by absorption paper, cotton-like pulp, a high-polymer water absorption material, etc., in combination. Right and left ends of the back sheet 11 are provided with a left notch 10a and a right notch 10b for forming left and right thigh openings 5a and 5b, respectively. Furthermore, as shown in FIG. 2, as for the trunks type diaper in this preferred embodiment, a distance L between opposite ends of a left edge 7a1 or a right edge 7b1 of a front main section 2 and a left edge 7a2 or a right edge 7b2 of a rear main section 3 is 30 cm or less. Preferably, it is 20 cm or less.

From a developed state of the diaper as shown in FIG. 2, the trunks type diaper is folded to form the front main section 2, the rear main section 3, and a crotch section 6, and arranged such that left and right edges 7a1 and 7b1 of the front main section 2 are mutually joined to left and right edges 7a2 and 7b2 of the rear main section 3 side by side, to form junction parts 8a and 8b in the right side and left side of the diaper 1 as shown in FIG. 1. The above-mentioned diaper 1 is provided with a waist opening 4 and the left and right thigh openings 5a and 5b in order that a user may put on and take off the diaper. FIG. 1 is a drawing in which this diaper 1 is viewed from the front.

Elastic members are provided for the diaper 1 in order to improve fitness of the diaper with respect to a body and in order to prevent the leakage of urine etc. In this preferred embodiment, a plurality of natural-rubber threads, synthetic-rubber threads, elastic non-woven fabrics, elastic film, etc. are arranged generally or partially as the elastic members. A plurality of elastic members are each arranged at the waist opening 4 peripheral edge, the front main section 2, the rear main section 3, peripheral edges of the left and right thigh openings 5a and 5b through the crotch section 6 in each circumferential direction. In FIGS. 1 through 3, three (for example) elastic members are arranged at the peripheral edge of the waist opening, three (for example) elastic members are each arranged at the front main section and the rear main section, and three (for example) elastic members are arranged at the peripheral edges of the left and right thigh openings through the crotch section. However, the number of the elastic members is of course arbitrarily chosen taking into consideration the body feeling of wearing and leakage prevention. In particular, for the sake of brevity, the drawings show a small number of elastic members which form body gathers 21 and are arranged at the front main section and the rear main section, but a larger number of them may actually be arranged. These elastic members respectively form waist gathers 20, body gathers 21, and left and right leg gathers 9a and 9b around the waist, the torso, and the thighs in each circumferential direction.

Further description will be carried out paying attention to the above-mentioned left and right leg gathers 9a and 9b. A plurality of the elastic members arranged at the left and right thigh openings 5a and 5b through the crotch section 6 are constituted by first and second thigh elastic members 15a and 15b. In a situation where each junction between the left or right edge 7a1 or 7b1 of the front main section 2 of the diaper and the left or right edge 7a2 or 7b2 of the rear main section 3 is separated to develop the diaper as shown in FIG. 2, the first thigh elastic member 15a extends substantially linearly from the left edge 7a1 of the front main section 2 to the right edge 7b1 of the front main section 2 via a front edge of the crotch section 6. On the other hand, the second thigh elastic member 15b extends substantially linearly from the left edge 7a2 of the rear main section 3 to the right edge 7b2 of the rear main section 3 via a rear edge of the crotch section 6. Unlike the thigh elastic member in accordance with the prior art, they are not arranged along front and rear side edges of the left and right thigh openings via the center of the crotch section.

In the first preferred embodiment in accordance with the present invention, as shown in FIG. 2 and described above, in a situation where the diaper is developed, the above-mentioned first and second thigh elastic members 15a and 15b are arranged substantially linearly across the width of the diaper. When the arrangement of these elastic members is viewed from the bottom, as shown in a bottom view of the diaper of FIG. 3, the first and second thigh elastic members 15a and 15b surround the left and right thigh openings 5a and 5b as one pair and are formed in the shape of a round. The left and right leg gathers 9a and 9b surround the pair of left and right thigh openings 5a and 5b in the shape of a round as a whole to hold both thighs as shown by arrow P.

On the other hand, in the diaper in accordance with the prior art, as described above, the thigh elastic members are arranged along the peripheral edges of thigh openings 54 to pass through the center of a crotch section 56, so that the left and right leg gathers 59a and 59b may separately surround peripheral edges of left and right thigh openings 55a and 55b in the shape of a ring to bring the thighs into close contact with the peripheral edges of the thigh opening 55a and 55b as shown by arrow P'.

Further description will be carried out with reference to FIG. 1, which shows an imaginary horizontal line A which passes through a junction point 17 between a left side edge 12a of the absorber 12 and the first thigh elastic member 15a, as well as an imaginary straight line C which passes through the above-mentioned junction point 17 and an intersection 18 between the first thigh elastic member 15a and the left edge 7a1 of the front main section 2. As a result, an angle X of the imaginary straight line C relative to the imaginary horizontal line A in the first preferred embodiment illustrated in FIG. 1 is set to 30 degrees or less, preferably 20 degrees or less. As described above, since an angle of imaginary straight line C' relative to an imaginary horizontal line A' of the diaper in accordance with the prior art is 45 degrees or more, the angle in accordance with the present invention is smaller than that in the prior art. The same applies to an angle between the imaginary horizontal line A which passes through a junction point, between a right side edge 12b of the absorber and the first thigh elastic member 15a, and an imaginary straight line passing through the above-mentioned junction point and an intersection between the first thigh elastic member 15a and the right edge 7b1 of the front main section.

Next, as to a second preferred embodiment, part different from the first preferred embodiment will be described with reference to FIGS. 4 and 5. The same description as that for the first preferred embodiment applies to the same part as that of the first preferred embodiment, which is given the same reference sign, so that the description will not be repeated.

The second preferred embodiment will be described paying attention to the left and right leg gathers 9a and 9b. A plurality of elastic members arranged at the left and right thigh openings 5a and 5b through the crotch section 6 are constituted by the first and second thigh elastic members 15a and 15b. In a situation where each junction between the left or right edge 7a1 or 7b1 of the front main section 2 of the diaper and the left or right edge 7a2 or 7b2 of the rear main section 3 is separated to develop the diaper as shown in FIG. 5, the first thigh elastic member 15a extends linearly from the left edge 7a1 of the front main section 2 to the right edge 7b1 of the front main section 2 via the front edge of the crotch section 6. On the other hand, the second thigh elastic member 15b extends linearly from the left edge 7a2 of the rear main section 3 to the right edge 7b2 of the rear main section 3 via the rear edge of the crotch section 6. Unlike the thigh elastic member in accordance with the prior art, they are not arranged along front and rear side edges of the left and right thigh openings via the center of the crotch section.

In the second preferred embodiment in accordance with the present invention, as shown in FIG. 5, in a situation where the diaper is developed, the above-mentioned first and second thigh elastic members 15a and 15b are arranged linearly across the width of the diaper. The first thigh elastic member 15a and the second thigh elastic member 15b are arranged in parallel with each other. When the arrangement of these elastic members is viewed from the bottom, as shown in the bottom view of the diaper of FIG. 3, the first and second thigh elastic members 15a and 15b surround the left and right thigh openings 5a and 5b as one pair and are formed in the shape of a round. The left and right leg gathers 9a and 9b surround the pair of left and right thigh openings 5a and 5b in the shape of a round as a whole to hold both thighs as shown by arrow P. In addition, although FIG. 3 is the bottom view of the diaper in the first preferred embodiment, the second preferred embodiment will be described with reference to FIG. 3 in this paragraph, since the bottom of the second preferred embodiment is also substantially the same.

Further description will be carried out with reference to FIG. 4, which shows the imaginary horizontal line A which passes through the junction point 17 between the left side edge 12a of the absorber 12 and the first thigh elastic member 15a, as well as the imaginary straight line C which passes through the above-mentioned junction point 17 and the intersection 18 between the first thigh elastic member 15a and the left edge 7a1 of the front main section 2. As a result, an angle of the imaginary straight line C relative to the imaginary horizontal line A in the second preferred embodiment illustrated in FIG. 4 is set to 0 degree (that is, the imaginary straight line C is arranged horizontally) . As described above, since the angle of imaginary straight line C', relative to the imaginary horizontal line A' of the diaper in accordance with the prior art is 45 degrees or more, the angle in accordance with the present invention is smaller than that in the prior art. The same applies to the angle between the imaginary horizontal line A which passes through the junction point, between the right side edge 12b of the absorber and the first thigh elastic member 15a, and the imaginary straight line passing through the above-mentioned junction point and the intersection between the first thigh elastic member 15a and the right edge 7b1 of the front main section.

Thirdly, as to a third preferred embodiment, part different from the first preferred embodiment will be described with reference to FIGS. 6 and 8. The same description as that for the first preferred embodiment applies to the same part as that of the first preferred embodiment, which is given the same reference sign, so that the description will not be repeated.

The third preferred embodiment will be described paying attention to the left and right leg gathers 9a and 9b. A plurality of elastic members arranged at the left and right thigh openings 5a and 5b through the crotch section 6 are constituted by the first and second thigh elastic members 15a and 15b. The first thigh elastic member 15a is constituted by a first thigh elastic member left part 15a1 and right part 15a2. The second thigh elastic member 15b is constituted by a second thigh elastic member left part 15b1 and right part 15b2. In a situation where each junction between the left or right edge 7a1 or 7b1 of the front main section 2 of the diaper and the left or right edge 7a2 or 7b2 of the rear main section 3 is separated to develop the diaper as shown in FIG. 6, the first thigh elastic member left part 15a1 extends from the left edge 7a1 of the front main section 2 to and beyond the left side edge 12a of the absorber 12, and the first thigh elastic member right part 15a2 extends from the right edge 7b1 of the front main section 2 to and beyond the right side edge 12b of the absorber, so that the first thigh elastic member 15a is arranged substantially linearly in the width direction. The second thigh elastic member left part 15b1 extends from the left edge 7a2 of the rear main section 3 to and beyond the left side edge 12a of the absorber, and the second thigh elastic member right part 15b2 extends from the right edge 7b2 of the rear main section 3 to and beyond the right side edge 12b of the absorber, so that the second thigh elastic member 15b is arranged substantially linearly in the width direction.

In the third preferred embodiment in accordance with the present invention, as shown in FIG. 6, in a situation where the diaper is developed, the above-mentioned first thigh elastic member left part 15a1 and right part 15a2 as well as the above-mentioned second thigh elastic member left part 15b1 and right part 15b2 are arranged substantially linearly across the width of the diaper. When the arrangement of these elastic members is viewed from the bottom, as shown in a bottom view of the diaper of FIG. 8, the first and second thigh elastic members 15a and 15b surround the left and right thigh openings 5a and 5b as one pair and are formed in the shape of a round. The left and right leg gathers 9a and 9b surround the pair of left and right thigh openings 5a and 5b in the shape of a round as a whole to hold both thighs as shown by arrow P.

As shown in FIG. 8, unlike the first preferred embodiment, in the third preferred embodiment, the left and right parts 15a1 and 15a2 of the first thigh elastic member are not continuous elastic members but separated at the crotch section 6 front edge. The left and right parts 15b1 and 15b2 of the second thigh elastic member are not continuous elastic members but separated at the crotch section 6 rear edge. However, as illustrated in FIG. 6, in a situation where the diaper is developed, the first thigh elastic member left part 15a1 extends from the left edge 7a1 of the front main section 2 to and beyond the left side edge 12a of the absorber 12, the first thigh elastic member right part 15a2 extends from the right edge 7b1 of the front main section 2 to and beyond the right side edge 12b of the absorber, the second thigh elastic member left part 15b1 extends from the left edge 7a2 of the rear main section 3 to and beyond the left side edge 12a of the absorber, and the second thigh elastic member right part 15b2 extends from the right edge 7b2 of the rear main section 3 to and beyond the right side edge 12b of the absorber. Therefore, the thigh elastic members 15a and 15b overlap a part of a junction area where the absorber 12 is joined to the back sheet 11. As with the first preferred embodiment, the left and right parts 15a1 and 15a2 of the first thigh elastic member and the left and right parts 15b1 and 15b2 of the second thigh elastic member surround the left and right thigh openings 5a and 5b as one pair and are formed in the shape of a round. The left and right leg gathers 9a and 9b surround the pair of left and right thigh openings 5a and 5b in the shape of a round as a whole to hold both thighs as shown by arrow P.

Fourthly, as to a fourth preferred embodiment, part different from the second preferred embodiment will be described with reference to FIG. 7. The same description as that for the second preferred embodiment applies to the same part as that of the second preferred embodiment, which is given the same reference sign, so that the description will not be repeated.

The fourth preferred embodiment will be described paying attention to the left and right leg gathers 9a and 9b. A plurality of elastic members arranged at the left and right thigh openings 5a and 5b through the crotch section 6 are constituted by the first and second thigh elastic members 15a and 15b. The first thigh elastic member 15a is constituted by the first thigh elastic member left part 15a1 and right part 15a2 . The second thigh elastic member 15b is constituted by the second thigh elastic member left part 15b1 and right part 15b2. In a situation where each junction between the left or right edge 7a1 or 7b1 of the front main section 2 of the diaper and the left or right edge 7a2 or 7b2 of the rear main section 3 is separated to develop the diaper as shown in FIG. 7, the first thigh elastic member left part 15a1 extends from the left edge 7a1 of the front main section 2 to and beyond the left side edge 12a of the absorber 12, and the first thigh elastic member right part 15a2 extends from the right edge 7b1 of the front main section 2 to and beyond the right side edge 12b of the absorber, so that the first thigh elastic member 15a is arranged linearly in the width direction. The second thigh elastic member left part 15b1 extends from the left edge 7a2 of the rear main section 3 to and beyond the left side edge 12a of the absorber, and the second thigh elastic member right part 15b2 extends from the right edge 7b2 of the rear main section 3 to and beyond the right side edge 12b of the absorber, so that the second thigh elastic member 15b is arranged linearly in the width direction.

In the fourth preferred embodiment in accordance with the present invention, as shown in FIG. 7, in a situation where the diaper is developed, the above-mentioned first thigh elastic member left part 15a1 and right part 15a2 as well as the above-mentioned second thigh elastic member left part 15b1 and right part 15b2 are arranged linearly across the width of the diaper. When the arrangement of these elastic members is viewed from the bottom, as shown in the bottom view of the diaper of FIG. 8, the first and second thigh elastic members 15a and 15b surround the left and right thigh openings 5a and 5b as one pair and are formed in the shape of a round. The left and right leg gathers 9a and 9b surround the pair of left and right thigh openings 5a and 5b in the shape of a round as a whole to hold both thighs as shown by arrow P. In addition, although FIG. 8 is the bottom view of the third preferred embodiment, the bottom of the fourth preferred embodiment is illustrated similarly to FIG. 8.

Unlike the second preferred embodiment, in the fourth preferred embodiment, the left and right parts 15a1 and 15a2 of the first thigh elastic member are not continuous elastic members but separated at the crotch section 6 front edge, as well as the left and right parts 15b1 and 15b2 of the second thigh elastic member are not continuous elastic members but separated at the crotch section 6 rear edge. However, as illustrated in FIG. 7, in a situation where the diaper is developed, the first thigh elastic member left part 15a1 extends from the left edge 7a1 of the front main section 2 to and beyond the left side edge 12a of the absorber 12, the first thigh elastic member right part 15a2 extends from the right edge 7b1 of the front main section 2 to and beyond the right side edge 12b of the absorber, the second thigh elastic member left part 15b1 extends from the left edge 7a2 of the rear main section 3 to and beyond the left side edge 12a of the absorber, and the second thigh elastic member right part 15b2 extends from the right edge 7b2 of the rear main section 3 to and beyond the right side edge 12b of the absorber. Therefore, the thigh elastic members 15a and 15b overlap a part of the junction area where the absorber 12 is joined to the back sheet 11. As with the second preferred embodiment, the left and right parts 15a1 and 15a2 of the first thigh elastic member and the left and right parts 15b1 and 15b2 of the second thigh elastic member surround the left and right thigh openings 5a and 5b as one pair and are formed in the shape of a round. The left and right leg gathers 9a and 9b surround the pair of left and right thigh openings 5a and 5b in the shape of a round as a whole to hold both thighs as shown by arrow P.

The trunks type diaper is arranged as in the first through fourth preferred embodiments above, so that the left and right leg gathers 9a and 9b may surround the left and right thigh openings 5a and 5b as one pair in the shape of a round to hold both thighs. Thus, a force which expands the absorber in a width direction is applied thereto. Further, although a shrinkage force of the thigh elastic members 15a and 15b acts along the above-mentioned imaginary straight line C, the angle of the above-mentioned imaginary straight line C relative to the above-mentioned imaginary horizontal line A is small as compared with the conventional one. Thus, a horizontal force M which is a component of the shrinkage force acting along the above-mentioned imaginary straight line C (that is, a horizontal force M towards the side of the diaper on the basis of the above-mentioned junction point 17 produced by the shrinkage force of the thigh elastic members 15a and 15b) becomes large. Accordingly, the force which expands the absorber 12 in the width direction becomes large, so that the absorber 12 is unlikely to be in the shape of a string or the shape of a strap. Further, an effect of preventing a gap between the thigh and a peripheral edge of the left or right thigh opening 5a or 5b at the crotch section 6 from taking place is improved as compared with the conventional one.

Furthermore, since the left and right thigh openings are surrounded in the shape of a round as one pair, the problem that it is hard to insert or pull out a leg when putting on or taking off the diaper is improved. In other words, since the left and right thigh openings are surrounded in the shape of a round as one pair, the left and right thigh openings are stretched out at the same time as the diaper is opened with both arms leftward and rightward. Therefore, unlike the prior art, it is possible to reduce the time and effort of inserting each leg into the diaper when putting the diaper on. In other words, as to the conventional diaper in which the left and right thigh openings shrink into the shape of a ring independently, it is impossible to provide the openings as required for inserting the thighs in the case of putting on the diaper unless the diaper is opened with both hands. However, according to the diaper in accordance with the present invention and to the above-described structure, the openings through which the legs are inserted can be obtained by a single hand by holding a part of the diaper (for example, part of leg gathers or absorber) in position. This means that it is possible for an old person with trouble in one hand etc. to change the diapers easily, which is considerable improvement, and provide an effect of increasing the opportunity to be able to put on the diaper alone and independently. Furthermore, since the left and right thigh openings are in the shape of a round as one pair, the thighs are not bound tight when wearing. Still further, there is no impression mark caused by tightening the elastic member etc., leading to good blood circulation of the thighs.

### Industrial Applicability

As described above, the trunks type diaper in accordance with the present invention has a good feeling of wearing around both thighs, stable absorptivity of bodily waste, and high leakage prevention, thus being useful for a trunks type diaper for an infant, an incontinent person, or an adult.

## Claims

1. A trunks type diaper which is constituted by a back sheet (11) and an absorber (12), wherein a front main section (2), a rear main section (3), a crotch section (6), a waist opening (4), and left and right thigh openings (5a, 5b) are formed, and wherein thigh elastic members are arranged at left and right thigh openings (5a, 5b) peripheral edges to form left and right leg gathers (9a, 9b), **characterized in that**
said thigh elastic members are constituted by first and second thigh elastic members (15a, 15b);
in a situation where each junction between left or right edge (7a1, 7b1) of said front main section (2) and left or right edge (7a2, 7b2) of said rear main section (3) is separated to develop the diaper, said first thigh elastic member (15a) extends substantially linearly from the left edge (7a1) of the front main section to the right edge (7b1) of the front main section via a crotch section (6) front edge; said second thigh elastic member (15b) also extends substantially linearly from the left edge (7a2) of the rear main section to the right edge (7b2) of the rear main section via a crotch section (6) rear edge;
in the bottom of said diaper in a situation where the left and right edges (7a1, 7b1) of said front main section (2) are joined to the left and right edges (7a2, 7b2) of said rear main section (3) to form the trunks type diaper, said first and second thigh elastic members are arranged to surround the left and right thigh openings (5a, 5b) as one pair; and
an angle (X) of an imaginary straight line (C) which passes through a junction point (17) and an intersection (18) between said first thigh elastic member (15a) and the left or right edge (7a1, 7b1) of the front main section relative to an imaginary horizontal line (A) which passes through said junction point (17) between left or right side edge (12a) of said absorber and said first thigh elastic member (15a) is set as 0 to 30 degrees.

2. A trunks type diaper which is constituted by a back sheet (11) and an absorber (12), wherein a front main section (2), a rear main section (3), a crotch section (6), a waist opening (4), and left and right thigh openings (5a, 5b) are formed, and wherein thigh elastic members are arranged at left and right thigh openings (5a, 5b) peripheral edges to form left and right leg gathers (9a, 9b), **characterized in that**
said thigh elastic members are constituted by first and second thigh elastic members (15a, 15b);
in a situation where each junction between left or right edge (7a1, 7b1) of said front main section (2) and left or right edge (7a2, 7b2) of said rear main section (3) is separated to develop the diaper, said first thigh elastic member (15a) extends linearly from the left edge (7a1) of the front main section to the right edge (7b1) of the front main section via a crotch section (6) front edge; said second thigh elastic member (15b) also extends linearly from the left edge (7a2) of the rear main section to the right edge (7b2) of the rear main section via a crotch section (6) rear edge;
in the bottom of said diaper in a situation where the left and right edges (7a1, 7b1) of said front main section (2) are joined to the left and right edges (7a2, 7b2) of said rear main section (3) to form the trunks type diaper, said first and second thigh elastic members are arranged to surround the left and right thigh openings (5a, 5b) as one pair; and
an angle (X) of an imaginary straight line (C) which passes through a junction point (17) and an intersection (18) between said first thigh elastic member (15a) and the left or right edge (7a1, 7b1) of the front main section relative to an imaginary horizontal line (A) which passes through said junction point (17) between the left or right side edge (12a) of said absorber and said first thigh elastic member (15a) is set to 0 degree.

3. A trunks type diaper which is constituted by a back sheet (11) and an absorber (12), wherein a front main section (2), a rear main section (3), a crotch section (6), a waist opening (4), and left and right thigh openings (5a, 5b) are formed, and wherein thigh elastic members are arranged at left and right thigh openings (5a, 5b) peripheral edges to form left and right leg gathers (9a, 9b), **characterized in that**
said thigh elastic members are constituted by first and second thigh elastic members (15a, 15b);
in a situation where each junction between left or right edge (7a1, 7b1) of said front main section (2) and left or right edge (7a2, 7b2) of said rear main section (3) is separated to develop the diaper, a left part (15a1) of said first thigh elastic member extends substantially linearly in the width direction from the left edge (7a1) of the front main section (2) to and beyond a left side edge (12a) of the absorber (12) ; a right part (15a2) of said first thigh elastic member extends substantially linearly in the width direction from the right edge (7b1) of the front main section (2) to and beyond a right side edge (12b) of the absorber; a left part (15b1) of said second thigh elastic member extends substantially linearly in the width direction from the left edge (7a2) of the rear main section (3) to and beyond the left side edge (12a) of the absorber; a right part (15b2) of said second thigh elastic member extends substantially linearly in the width direction from the right edge (7b2) of the rear main section (3) to and beyond the right side edge (12b) of the absorber;
in the bottom of said diaper in a situation where the left and right edges (7a1, 7b1) of said front main section (2) are joined to the left and right edges (7a2, 7b2) of said rear main section (3) to form the trunks type diaper, said first and second thigh elastic members are arranged to surround the left and right thigh openings (5a, 5b) as one pair; and
an angle (X) of an imaginary straight line (C) which passes through a unction point (17) and an intersection (18) between said first thigh elastic member (15a) and the left or right edge (7a1, 7b1) of the front main section relative to an imaginary horizontal line (A) which passes through said junction point (17) between the left or right side edge (12a, 12b) of said absorber and said first thigh elastic member (15a) is set as 0 to 30 degrees.

4. A trunks type diaper which is constituted by a back sheet (11) and an absorber (12), wherein a front main section (2), a rear main section (3), a crotch section (6), a waist opening (4), and left and right thigh openings (5a, 5b) are formed, and wherein thigh elastic members are arranged at left and right thigh openings (5a, 5b) peripheral edges to form left and right leg gathers (9a, 9b), **characterized in that**
said thigh elastic members are constituted by first and second thigh elastic members (15a, 15b);
in a situation where each junction between left or right edge (7a1, 7b1) of said front main section (2) and left or right edge (7a2, 7b2) of said rear main section (3) is separated to develop the diaper, a left part (15a1) of said first thigh elastic member extends linearly in the width direction from the left edge (7a1) of the front main section (2) to and beyond a left side edge (12a) of the absorber (12) ; a right part (15a2) of said first thigh elastic member extends linearly in the width direction from the right edge (7b1) of the front main section (2) to and beyond a right side edge (12b) of the absorber; a left part (15b1) of said second thigh elastic member extends linearly in the width direction from the left edge (7a2) of the rear main section (3) to and beyond the left side edge (12a) of the absorber; a right part (15b2) of said second thigh elastic member extends linearly in the width direction from the right edge (7b2) of the rear main section (3) to and beyond the right side edge (12b) of the absorber;
in the bottom of said diaper in a situation where the left and right edges (7a1, 7b1) of said front main section (2) are joined to the left and right edges (7a2, 7b2) of said rear main section (3) to form the trunks type diaper, said first and second thigh elastic members are arranged to surround the left and right thigh openings (5a, 5b) as one pair; and
an angle (X) of an imaginary straight line (C) which passes through a junction point (17) and an intersection (18) between said first thigh elastic member (15a) and the left or right edge (7a1, 7b1) of the front main section relative to an imaginary horizontal line (A) which passes through said junction point (17) between the left or right side edge (12a, 12b) of said absorber and said first thigh elastic member (15a) is set to 0 degree.
